# EUROPEAN PATENT APPLICATION

(11) **EP 4 230 224 A1**
(43) Date of publication of application: **23.08.2023**
(21) Application number: 21878872.7
(22) Date of filing: 05.02.2021
(51) Int. Cl.: A61K 47/64, A61K 47/54, A61K 47/69, A61K 31/427, A61K 31/5365, A61K 38/07, A61P 35/00

(54) **AFFIBODY-CYTOTOXIN CONJUGATE USED FOR ACTIVE TUMOR TARGETING THERAPY, AND NANOPARTICLES, PREPARATION METHOD, AND APPLICATION THEREOF**

(30) Priority: 16.10.2020 CN 202011114498
(71) Applicant: SHANGHAI JIAOTONG UNIVERSITY, Minhang District Shanghai 200240 (CN)
(72) Inventor: YAN, Deyue, Shanghai 200240 (CN); XIA, Xiaoxia, Shanghai 200240 (CN); XIA, Xuelin, Shanghai 200240 (CN); YANG, Xiaoyuan, Shanghai 200240 (CN); HUANG, Wei, Shanghai 200240 (CN)
(74) Representative: Kransell & Wennborg KB
(86) International application number: PCT/CN2021/075469
(87) International publication number: WO 2022/077815

(57) **Abstract**

The invention discloses a conjugate for active targeted therapy of tumors which is obtained by coupling a hydrophilic affibody with an active tumor targeting function and a hydrophobic anti-tumor cytotoxin through a small molecule linker. The affibody has a specific binding activity and a high affinity to receptors overexpressed on surfaces of various tumor cells, and the cytotoxin is a highly effective anti-tumor drug. The invention further discloses a nanoparticle thereof, a preparation method therefor and an application thereof. The affibody-cytotoxin conjugate involved in the invention can self-assemble in water to form the nanoparticle with the affinity body as a shell and the cytotoxin as an inner core. Since the affibody can specifically recognize and bind to receptors overexpressed on the surfaces of the tumor cells, the nanoparticle can be efficiently enriched at tumor sites and biodegraded through the linker to release the cytotoxin and reach effective therapeutic concentrations for inhibiting the growth of the tumors while reducing toxic and side effects to normal organs and tissues, so as to provide a new solution for active targeted precision therapy of tumors.

## Description

### FIELD OF THE INVENTION

The invention relates to the technical field of biology and nanomedicine, in particular, to a preparation method for the nanoparticle of the affibody-cytotoxin conjugate for active targeted therapy of tumors and an application thereof.

### BACKGROUND OF THE INVENTION

Malignant tumor (cancer) is a major disease that seriously threatens human survival and social development, and has become a difficult problem and a major challenge that needs to be solved urgently all over the world. At present, chemotherapy remains the mainstay of cancer treatment. Small molecule cytotoxins have the advantages of clear structure, high permeability, and strong cytotoxicity. However, due to the small molecular weight and lack of targeting specificity, the small molecule cytotoxin will be rapidly metabolized by the body after administration, and often causes severe toxic side effects in the body, so the clinical application is limited.

Antibody-drug conjugate (ADC) technology, which combines the target selectivity of monoclonal antibodies and the high efficiency of small molecule cytotoxins, provides a new method for "precision therapy" of tumors. As a novel class of tumor therapeutic drugs, the research and development of ADC has not only received extensive attention, but also the products have been used clinically. However, the existing ADC drugs still have many shortcomings as follows: (1) the molecular weight of the antibody is too large (1.5×10⁵ Da), resulting in a low penetration rate of the drug inside the tumor; (2) the number of coupling sites between the antibody surface and small molecule cytotoxins is uncertain, resulting in inconsistent drug loading rates between batches, making it difficult to obtain uniform products; (3) there is a lack of high-quality targets and more ideal antibodies to further increase the endocytosis rate and antibody-mediated biological effects. Therefore, how to overcome the above shortcomings and the development of ADC drugs with high drug delivery ratio and uniform cytotoxin carrying capability has become imminent. Based on this, trying to use other targeting carriers with stronger affinity, higher selectivity for tumor tissues or cells, and uniform binding to cytotoxins for developing new active targeted anti-tumor drugs is not only necessary, but also has great scientific significance and clinical application value.

### BRIEF SUMMARY OF THE DISCLOSURE

The invention provides an affibody-cytotoxin conjugate for active targeted therapy of tumors, which may solve the above drawbacks in the prior art.

The technical solution of the invention is as follows:
A affibody-cytotoxin conjugate for active targeted therapy of tumors mainly includes a cytotoxin and an affibody with active targeting function, wherein the conjugate is obtained by covalent coupling the cytotoxin and the affibody through a small molecule linker; 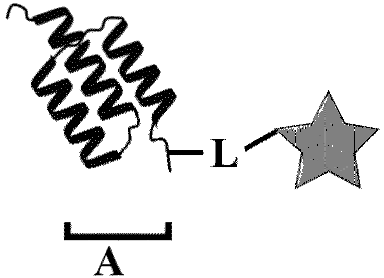
wherein A is the affibody, L is the organic small molecule linker, and is the cytotoxin.

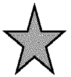

Preferably, the affibody is selected from at least one of an affibody targeting human epithelial growth factor receptors (HER) Z_{HER2:342} or Z_{EGFR:1907}, an affibody targeting human insulin-like growth factor receptors Z_{IGF1R:4551}, and an affibody targeting human platelet-derived growth factor receptors β Z_{PDGFRβ:07591}, amino acid sequences thereof being in the following formula (II~V):

HMCVDNKFNKEMRNAYWEIALLPNLNNQQKRAFIRSLYDDPSQS
ANLLAEAKKLNDAQAPK (II)

MGHHHHHHHHHHSSGHIDDDDKHM VDNKFNKEMWAAWEEIRN
LPNLNGWQMTAFIASLVDDPSQSANLLAEAKKLNDAQAPK C (III)

MGHHHHHHHHHHSSGHIDDDDKHMAENKFNKEGFYAALEILIPN
LTQKQRGAFISSLSDDPSQSANLLAEAKKLNDAQGGGC (IV)

MGHHHHHHHHHHSSGHI DDDDKHMAENKFNKELIEAAAEIDALP

NLNRRQWNAFIKSLVDDPSQSSELLSEAKKLNDSQAPKC (V)

Preferably, the cytotoxin is selected from at least one of epothilone B and derivatives thereof, maytansine and derivatives thereof, and monomethyl auristatin E (MMAE) and derivatives thereof.

Preferably, the small molecule linker is a linker with a maleimide-terminated linker, and the small molecule linker is connected to a sulfhydryl group of the affibody through a maleimide group.

Preferably, the small molecule linker is selected from at least one of formula (II), formula (III) and formula (IV) as below; 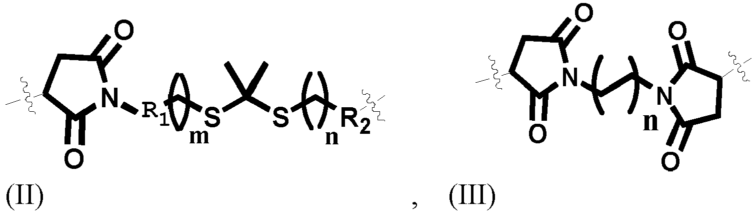 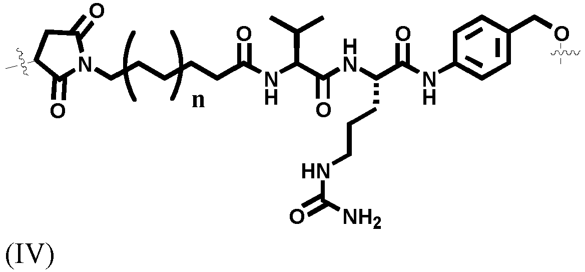
wherein R1 is selected from -alkyl-C(O)NH-, -alkyl-SO2NH-, or C1-8 alkoxy; R2 is selected from carboxyl or acid chloride, and n and m are each independently selected from 2 to 10;
the alkyl is selected from C1-8 alkyl; the alkyl group and alkoxy group are unsubstituted or substituted by 1, 2, 3 substituents selected from the group consisting of halogen, cyano, acetyl, hydroxy, hydroxymethyl, hydroxyethyl, carboxyl, C1-8 alkyl, C1-8 alkoxy, halogenated C1-8 alkyl, C3-8 cycloalkyl, C3-8 cycloalkoxy, Halogenated C1-8 alkoxy, -C(O)C1-10 alkyl, -C(O)OC1-10 alkyl, -OC(O)C1-10 alkyl, -CONRa0Rb0; Ra0 and Rb0 are each independently hydrogen or C1-8 alkyl.

A nanoparticle of the affibody-cytotoxin conjugate for active targeted therapy of tumors is obtained by self-assembly of the conjugate according to any one of the above items in an aqueous medium.

Preferably, a shell of the nanoparticle is composed of the affibody with active targeting function, an inner core of the nanoparticle is composed of the small molecule linker and the cytotoxin, and a particle size of the nanoparticle is less than 200 nm.

A preparation method for the nanoparticle of the affibody-cytotoxin conjugate for active targeted therapy of tumors includes steps of: (1) bonding the hydrophobic cytotoxin containing hydroxyl and/or amino and/or sulfhydryl to the small molecule linker by way of such as esterification, click and other chemical reactions, which is further subjected to conjugation reaction with an active targeting affibody, so as to obtain the affibody-cytotoxin conjugate with active targeting function;
(2) dissolving a certain amount of the affibody-cytotoxin conjugate in the step (1) in a certain volume of an organic solvent, which is dripped into slowly-stirring ultrapure water at room temperature, and removing the organic solvent, so as to obtain a nanoparticle aqueous solution of the conjugate with active targeting function.

Preferably, the organic solvent in the step (2) is selected from at least one of dimethyl sulfoxide, N,N'-dimethylformamide, ethanol, and isopropanol.

An application of the affibody-cytotoxin conjugate for active targeted therapy of tumors according to any one of the above items and the nanoparticle prepared by the preparation method according to any one of the above items in preparing drugs for active targeted therapy of tumors.

Compared with the conventional art, the present invention has the following beneficial effects:
The invention discloses a conjugate for active targeted therapy of tumors which is obtained by coupling a hydrophilic affibody with an active tumor targeting function and a hydrophobic anti-tumor cytotoxin through a small molecule linker. The affibody is a helical structure containing 58 amino acid residues and 3 α, and is a single-chain polypeptide molecule with a high affinity and a specific binding activity to receptors overexpressed on surfaces of various tumor cells.

The affibody-cytotoxin conjugate with active targeting function of the invention may self-assemble in water to form the nanoparticle and has a shell that is the affibody with active targeting function, which may bind highly specifically to receptors overexpressed on the surfaces of tumor cells, so as to enhance the enrichment of drugs in tumor sites, make drugs enter tumor cells more efficiently, and reduce toxic side effects on normal cells and tissues; when the conjugate nanoparticle enters the tumor cells, the thioketal bond or amide bond in the organic small molecule linker is degraded to release the cytotoxin and kill the tumor cells, thereby reducing the toxic side effects on normal cells and achieving the purpose of targeted therapy for the cancer.

Certainly, any one product for implementing the present invention is unnecessary to achieve all the above advantages at the same time.

### Brief Description of the Drawings

Fig. 1 is a Maldi-Tof-MS spectrum of a conjugate Z_{HER2:342}-Epo B with active targeting function according to Embodiment 1;
Fig. 2 is a transmission electron microscope photo of a nanoparticle of the conjugate Z_{HER2:342}-Epo B with active targeting function according to Embodiment 1;
Fig. 3 is a dynamic light scattering data plot of the nanoparticle of the conjugate Z_{HER2:342}-Epo B with active targeting function according to Embodiment 1;
Fig. 4 is a high-resolution mass spectrogram of an intermediate B-1 obtained according to Embodiment 2;
Fig. 5 is a Maldi-Tof-MS spectrum of a conjugate Z_{HER2:342}-DM1 with active targeting function according to Embodiment 2;
Fig. 6 is a Maldi-Tof-MS spectrum of a conjugate Z_{HER2:342}-MMAE with active targeting function according to Embodiment 3;
Fig. 7 is a Maldi-Tof-MS spectrum of a conjugate Z_{EGFR:1907}-DM1 with active targeting function according to Embodiment 4;
Fig. 8 is a Maldi-Tof-MS spectrum of a conjugate Z_{EGFR:1907}-MMAE with active targeting function according to Embodiment 5;
Fig. 9 is a Maldi-Tof-MS spectrum of a conjugate Z_{IGF1R:4551}-DM1 with active targeting function according to Embodiment 6;
Fig. 10 is a Maldi-Tof-MS spectrum of a conjugate Z_{IGF1R:4551}-MMAE with active targeting function according to Embodiment 7;
Fig. 11 is a Maldi-Tof-MS spectrum of a conjugate Z_{PDGFRβ:07591}-DM1 with active targeting function according to Embodiment 8;
Fig. 12 is a Maldi-Tof-MS spectrum of a conjugate Z_{PDGFRβ:07591}-MMAE with active targeting function according to Embodiment 9;
Fig. 13 is a diagram showing the inhibitory effect of a conjugate nanoparticle with active targeting function on tumor cell growth according Embodiment 10;
Fig. 14 is a pharmacokinetic diagram of the nanoparticle of the conjugate Z_{HER2:342}-Epo B with active targeting function in SD rats according to Embodiment 11;
Fig. 15 is a diagram of long-term cycle imaging in SKOV-3 tumor-bearing nude mice of the nanoparticle of the conjugate Z_{HER2:342}-Epo B with active targeting function after fluorescent modification according to Embodiment 12;
Fig. 16 is a curve showing changes in tumor volume of tumor-bearing mice treated with the nanoparticle of the conjugate Z_{HER2:342}-MMAE with active targeting function according to Embodiment 13;
Fig. 17 is a curve showing changes in body weight of tumor-bearing mice treated with the nanoparticle of the conjugate Z_{HER2:342}-MMAE with active targeting function according to Embodiment 13;
Fig. 18 is a diagram showing therapeutic effects of tumor-bearing mice treated with the nanoparticle of the conjugate Z_{HER2:342}-MMAE with active targeting function according to Embodiment 13.

### Detailed Description of the Invention

The invention provides an affibody-cytotoxin conjugate for active targeted therapy of tumors, which mainly includes a cytotoxin and an affibody with active targeting function, wherein the conjugate is obtained by covalent coupling the cytotoxin and the affibody through a small molecule linker.

Affibody is a new type of affinity ligand and has an area of the molecule mediating the receptor to bind reaching 8nm², which is similar to the area of the binding region caused by the antigen-antibody reaction. Its relative molecular mass is small and its structure is stable, so it is called "artificial antibody". These unique properties make it have broad application prospects in the fields of biology and immunology.

The affibody consists of 58 amino acid residues, with a relative molecular mass of about 6.5 × 10³Da, and is a single-chain polypeptide molecule with three α-helical structures. The affibody not only functions similar to the antibody, but also has the following advantages that the antibody do not have: (1) The relative molecular mass is small, which is only 1/25 of the antibody molecule, and it has very high stability ans is easy to prepare. (2) It has high selectivity and affinity, and its affinity may reach up to 22 pmol/L. (3) The amino acid sequence does not contain cysteine, and a cysteine may be directionally introduced when the affibody molecule is prepared, which is then connected to a payload through the reaction of thiol and other active groups. (4) It can be quickly located at the site of action, and has good tissue penetration, and may reach target sites that some antibody molecules may not reach.

At present, the application fields of the affibody are expanding day by day, but there are still relatively few research reports on the affibody-drug coupling. The unique advantages of the affibody determine that it can be used as high-affinity targeting molecules linked to anti-tumor drugs to achieve active targeted therapy of tumors.

In the prior art, there are examples of coupling to cytotoxins via cell-binding agents; the number of amino acids containing 2-hydroxyethylamine groups on the cell-binding agent is multiple; due to the limitation of chemical reactivity, the aldehyde groups obtained by oxidation on each cell-binding agent molecule cannot be combined with the cytotoxic agent in the end, resulting in an indeterminate number of cytotoxic agent molecules carried by each cell-binding agent molecule; this defect will inevitably cause batch-to-batch variability in the final product, and it is also an area where current antibody-drug conjugate (ADC) drugs urgently need to be improved.

In the invention, an affibody-cytotoxin conjugate is prepared with the affibody as a targeting carrier. First, since the affibody molecule itself does not contain cysteine, a cysteine molecule is introduced into the amino acid sequence of the affibody, and at the same time maleimidation modification is performed on the cytotoxin; then, through the click reaction between cysteine and maleimide, the two are coupled to obtain the affibody-cytotoxin conjugate. In this type of conjugate, one affibody molecule is only combined with one cytotoxin molecule, which avoids the instability of ADC drug-loading ratio and large batch-to-batch variation. Meanwhile, based on the amphiphilic molecular structure of the conjugate, it may self-assemble into the nanoparticle in water. This strategy not only endows the cytotoxin with excellent active targeting function, but also endows the nanoparticle of conjugate with the characteristics of passively being targeted to be enriched on the tumor site (EPR effect). The high-efficiency targeting property effectively reduces the toxic side effects of the carried cytotoxins on the body, and the nanometerization feature effectively improves the biological half-life period of the conjugate in vivo, so it has good clinical application prospects and huge market value. Since the affibody can specifically recognize and bind to receptors overexpressed on the surfaces of the tumor cells, the nanoparticle prepared thereby can be efficiently enriched at tumor sites and biodegraded through the linker to release the cytotoxin and reach effective therapeutic concentrations for inhibiting the growth of the tumors while reducing toxic and side effects to normal organs and tissues, so as to provide a new solution for active targeted precision therapy of tumors. To sum up, the present invention also provides a preparation and application of efficient tumor-targeted nanomedicines self-assembled from affibody-cytotoxin conjugates for cancer therapy.

An affibody targeting human epithelial growth factor receptors (HER) Z_{HER2:342} or Z_{EGFR:1907}, an affibody targeting human insulin-like growth factor receptors Z_{IGF1R:4551}, and an affibody targeting human platelet-derived growth factor receptors β Z_{PDGFRβ:07591} and so on are all prepared by the method of biological fermentation of Escherichia coli combined with induced expression. For example, for the affibody ZHER2:342, the corresponding DNA base sequence is prepared according to the amino acid sequence of the affibody, and connected to a plasmid. Then the recombinant plasmid is transformed into the E. coli BL21 (DE3) strain, and the E. coli strain carrying the target affinity body Z_{HER2:342} gene is obtained after screening for specific plasmid resistance. And then an appropriate amount of the strain is taken and placed in a 2 L large culture bottle for expansion. After it grows to a certain concentration, an inducer is added to induce expression for 12 h for collecting the bacteria. A high-pressure homogenizer is used to break up the bacterial cells, which is centrifuged to get a supernatant, and the supernatant is separated and purified through a Ni-NTA affinity chromatography column, so as to obtain an affibody Z_{HER2:342} eluate; the eluate of the obtained affibody protein is dialyzed to remove salt (the molecular weight cut-off of the dialysis bag is 3.5 kDa), concentrated by centrifugation, and then freeze-dried to obtain the cotton-like affibody Z_{HER2:342} protein, which is sealed and stored.

In the description, a range represented by "one value to another value" is a way to avoid listing in the specification a summary representation of all values in that range. Therefore, a record of a particular range of values covers any number within that range and a small range of values defined by any number within that range, which is the same as the arbitrary value and the smaller range of values written clearly in the specification.

The following further describes the present invention in combination with specific embodiments. It should be understood that these embodiments are only used for illustrating the invention, but not for the limitation of the scope of the invention. Improvements and adjustments made by those skilled in the art according to the invention in practical applications still belong to the protection scope of the invention.

### Embodiment 1

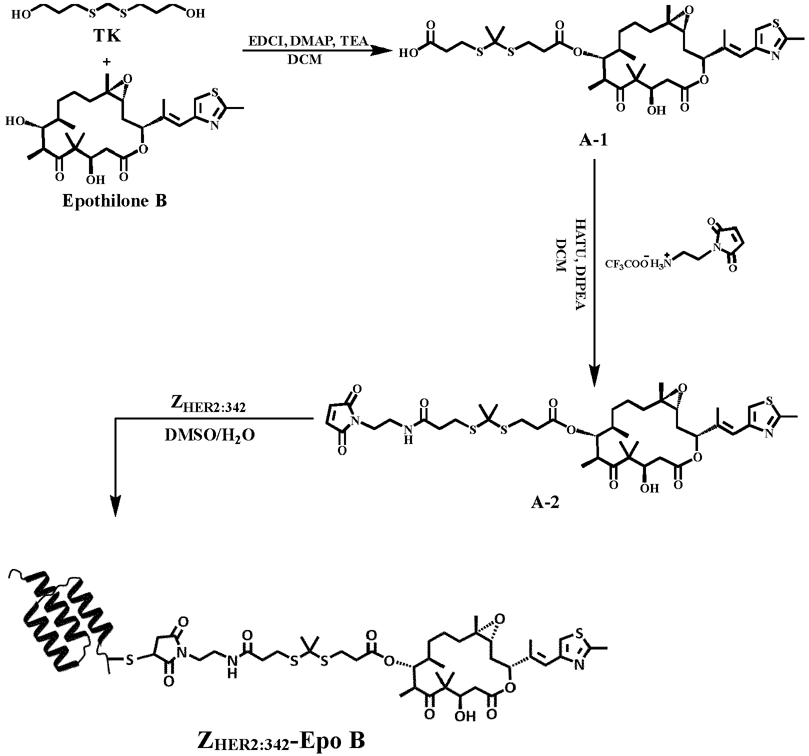

### 1.1 Synthesis of Intermediate A-1

3,3'- (Propane-2,2-diylbis (sulfanediyl))dipropionic acid (TK, 252.05mg), 1-ethyl-(3-dimethylaminopropyl)carbodiimide hydrochloride (EDCI, 287.55mg), 4-dimethylaminopyridine (DMAP, 12.2mg) and anhydrous triethylamine (TEA, 0.28mL) are added into a reaction flask and then 25mL anhydrous dichloromethane is added, which are stirred for reaction for 1 hour at room temperature; then epothilone B (507.27mg) is added to stir for continuous reaction for 24 hours at room temperature. After the reaction, 20 mL deionized water is added for extraction to collect an organic phase, and then a white powder intermediate A-1 (345.92 mg, yield of 46.6%) is obtained by column chromatography using a mixed solution of dichloromethane and methanol with a volume ratio of (20:1) as an eluent. The assignment of each proton peak in an NMR spectrum of ¹H of the intermediate A-1 is as follows: δ (ppm from TMS), ¹H NMR (CDCl₃,400 MHz) *δ* 7.04 (1H, s), 6.82 (1H, bs), 5.34 (1H, dd), 5.26 (1H, dd), 4.21 (1H, m), 4.19 (1H, bs), 3.56 (1H, dq), 2.93 (4H, m), 2.84 (1H, dd), 2.75 (3H, s), 2.69 (4H, m), 2.51 (1H, dd), 2.35 (1H, dd), 2.16 (1H, ddd), 2.12 (3H,d), 1.95 (1H, ddd), 1.74 (2H, m), 1.63 (6H, s), 1.35 (2H, m), 1.32 (3H, m), 1.30 (3H, s), 1.27 (3H, s), 1.16 (3H, d), 1.13 (3H, s), 0.97 (3H, d). HRMS (Esi-TOF, m/z) m/z [M+H]⁺ calcd: C₃₆H₅₅NO₉S₃: 742.3039, found: 742.3122.

### 1.2 Synthesis of Intermediate A-2

The intermediate A-1 (74.13mg), N-(2-aminoethyl)maleimide trifluoroacetate (38.12mg), 2-(7-benzotriazole oxide)-N,N,N',N'-tetramethyluronium hexafluorophosphate (HATU, 57.04mg) and N,N-diisopropylethylamine (DIPEA, 42µL) are added in a reaction flask, and then 20mL anhydrous dichloromethane is added, which are stirred for reaction for 6 hours at room temperature. After the reaction, 20 mL deionized water is added for extraction to collect an organic phase, and then a white powder intermediate A-2 (74.07 mg, yield of 85.7%) is obtained by column chromatography using a mixed solution of dichloromethane and methanol with a volume ratio of (20:1) as an eluent.

The assignment of each proton peak in an NMR spectrum of ¹H of the intermediate A-2 obtained in the embodiment is as follows: δ (ppm from TMS), ¹H NMR (CDCl₃, 400 MHz) 7.01 (1H, s), 6.74 (1H, s), 6.64 (1H, bs), 5.49 (1H, dd ), 5.33(1H, dd ), 4.28 (1H, bs), 4.12 (1H, m), 3.75 (1H, m), 3.71 (2H, t), 3.50 (2H, t), 3.20 (1H, dq), 2.90 (4H, m), 2.84 (1H, dd), 2.72 (3H, s), 2.67 (2H, t), 2.58 (1H, dd), 2.49 (1H, dd), 2.44 (2H, t), 2.18 (1H, d), 2.12 (3H, d), 1.97 (1H, d), 1.68 (2H, m), 1.60 (6H, s). 1.47 (2H, m), 1.44 (3H, m), 1.38 (3H, s), 1.29 (3H, s), 1.16 (3H, d), 1.09 (3H, s), 0.94 (3H, d).

### 1.3 Synthesis of Conjugate Z_{HER2:342}-Epo B

The affibody Z_{HER2:342} (10mg) is dissolved in 5mL PBS solution, and then the intermediate A-2 (1.22mg) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{HER2:342}-Epo B after the dialysis is completed.

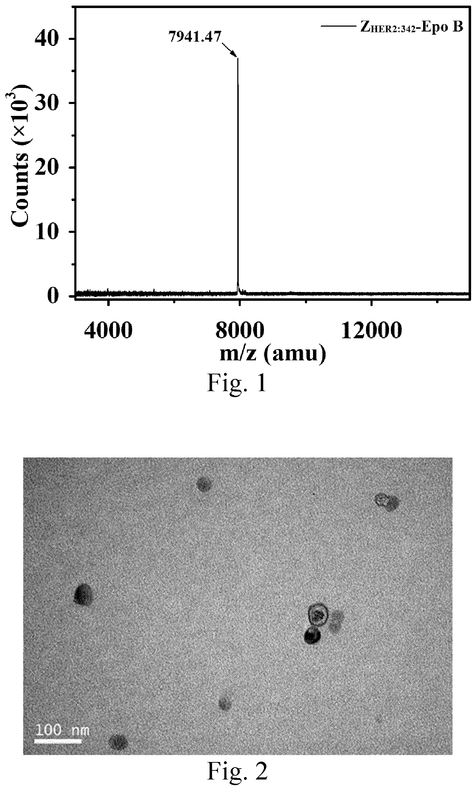

The data for Maldi-Tof-MS spectrum of the conjugate Z_{HER2:342}-Epo B prepared by the embodiment are shown in Fig. 1, indicating that the single peak of the molecular weight of the product appears at 7941.47, which is basically consistent with the theoretical molecular weight of 7942.21.

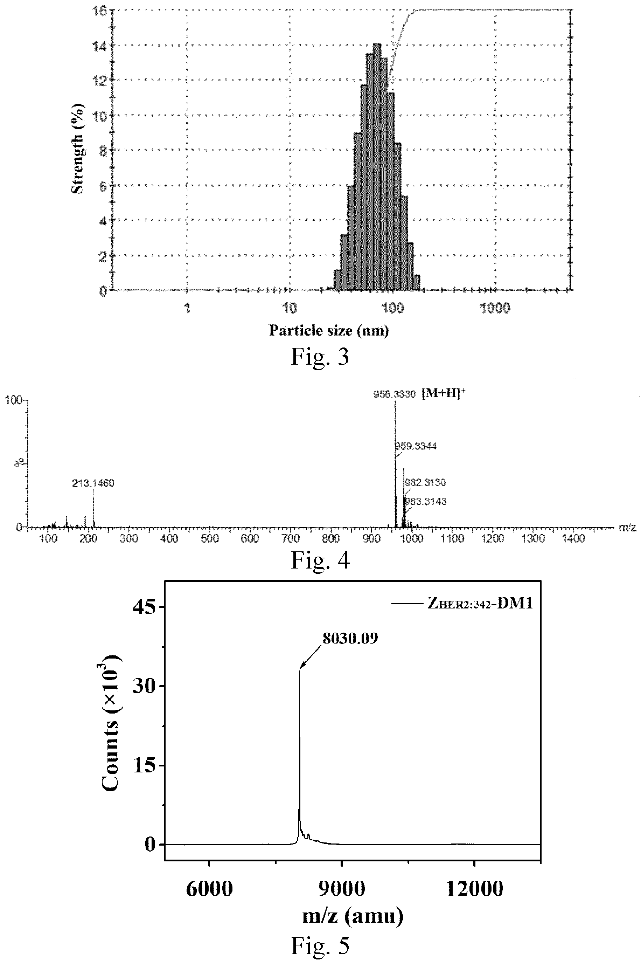

The conjugate Z_{HER2:342}-Epo B prepared by the above is dissolved in dimethyl sulfoxide, and dripped into water at room temperature while removing dimethyl sulfoxide, so as to obtain a nanoparticle aqueous solution of the conjugate. A transmission electron microscope photo of the anti-tumor nanoparticle prepared based on the conjugate Z_{HER2:342}-Epo B in the embodiment is shown in Fig. 2, and an average particle size of nanoparticle is around 100 nm; dynamic light scattering data are shown in Fig. 3, an average particle size of nanoparticle is around 100 nm, which is basically consistent with the data of the transmission electron microscope photo.

The anti-tumor nanoparticle of Z_{HER2:342}-Epo B in the embodiment has good uniformity, which may effectively reduce the batch-to-batch difference in the preparation of the nanoparticle and improve the stability in quality; secondly, good uniformity may ensure that the drug nanoparticle has consistent pharmacokinetic behavior and metabolic pathways in the body when circulating in the body, which will facilitate passing clinical evaluation.

### Embodiment 2

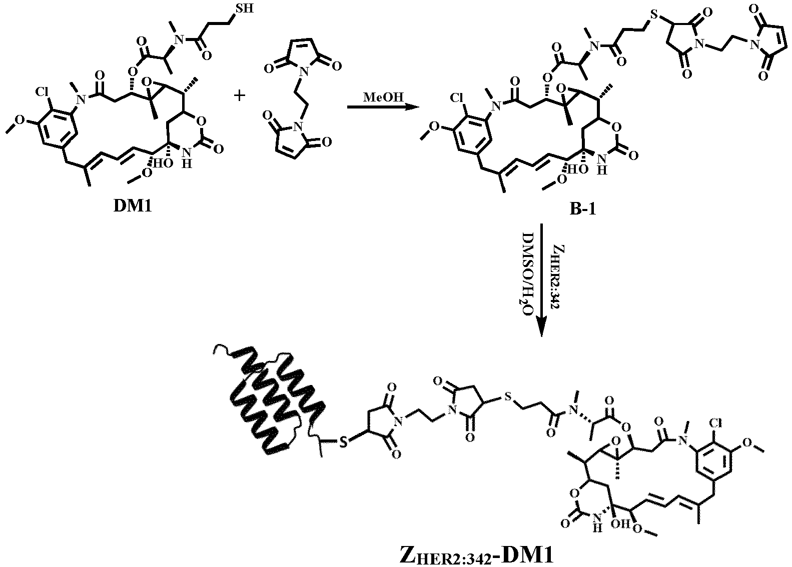

### 2.1 Synthesis of Intermediate B-1

N2'-deacetyl-N2'-(3-Mercapto-1-oxopropyl)maytansine (DM1, 737.2mg) and ethylbismaleimide (1.1g) are added in a reaction flask, and then 50 mL methanol is added, which are stirred for reaction for 12 hours at room temperature. After the reaction, rotary evaporation and concentration are performed, and a light yellow powder intermediate B-1 (883.4mg, yield of 92.3%) is obtained by column chromatography using a mixed solution of dichloromethane and methanol with a volume ratio of (20:1) as an eluent.

A mass spectrum of the intermediate B-1 prepared in the embodiment is shown in Fig. 4, wherein HRMS (Esi-TOF, m/z)m/z [M+H]⁺ calcd: C₄₅H₅₆ClN₅O₁₄S: 958.3233, found: 958.3330.

### 2.2 Synthesis of Conjugate Z_{HER2:342}-DM1

The affibody Z_{HER2:342} (10mg) is dissolved in 5mL PBS solution, and then the intermediate B-1 (1.35 mg) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{HER2:342}-DM1 after the dialysis is completed.

The Maldi-Tof-MS spectrum of the conjugate Z_{HER2:342}-DM1 prepared by the embodiment are shown in Fig. 5, indicating that the single peak of the molecular weight of the product appears at 8030.09, which is basically consistent with the theoretical molecular weight of 8038.25.

The conjugate Z_{HER2:342}-DM1 prepared by the above is dissolved in dimethyl sulfoxide, and dripped into water at room temperature while removing dimethyl sulfoxide, so as to obtain a nanoparticle aqueous solution of the conjugate. An average size of the anti-tumor nanoparticle based on the conjugate Z_{HER2:342}-DM1 prepared in the embodiment is around 100nm.

### Embodiment 3

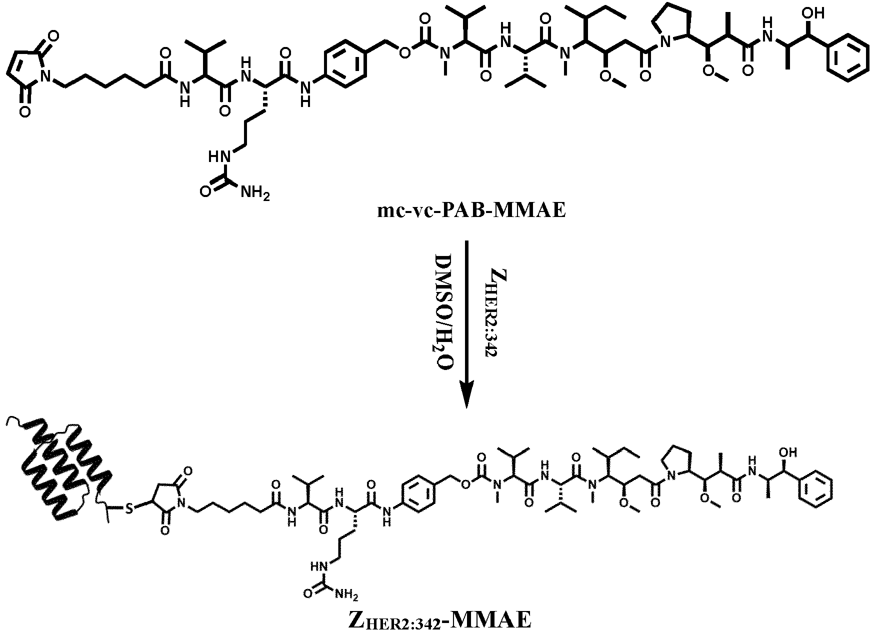

### 3.1 Synthesis of Conjugate Z_{HER2:342}-MMAE

The affibody Z_{HER2:342} (10mg) is dissolved in 5mL PBS solution, and then an intermediate mc-vc-PAB-MMAE (1.85 mg, purchased from Nanjing Chemlin Biomedical Technology Co., Ltd.) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{HER2:342}-MMAE after the dialysis is completed.

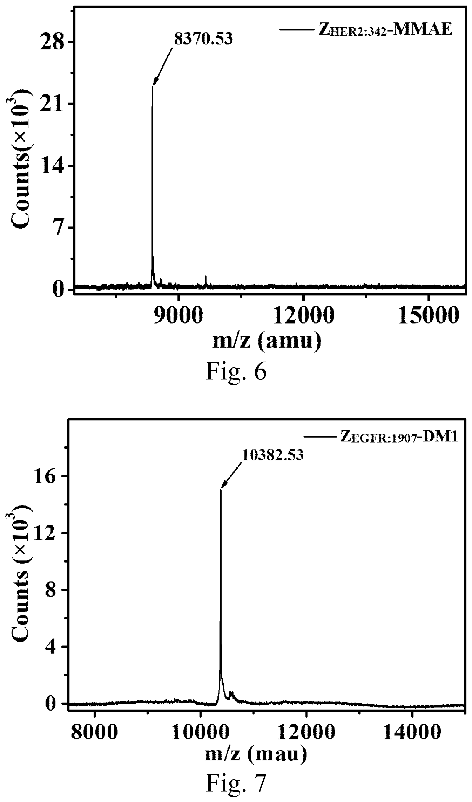

The Maldi-Tof-MS spectrum of the conjugate Z_{HER2:342}-MMAE prepared by the embodiment are shown in Fig. 6, indicating that the single peak of the molecular weight of the product appears at 8370.53, which is basically consistent with the theoretical molecular weight of 8393.63.

The conjugate Z_{HER2:342}-MMAE prepared by the above is dissolved in dimethyl sulfoxide, and dripped into water at room temperature while removing dimethyl sulfoxide, so as to obtain a nanoparticle aqueous solution of the conjugate. An average size of the anti-tumor nanoparticle based on the conjugate Z_{HER2:342}-MMAE prepared in the embodiment is around 100nm.

### Embodiment 4

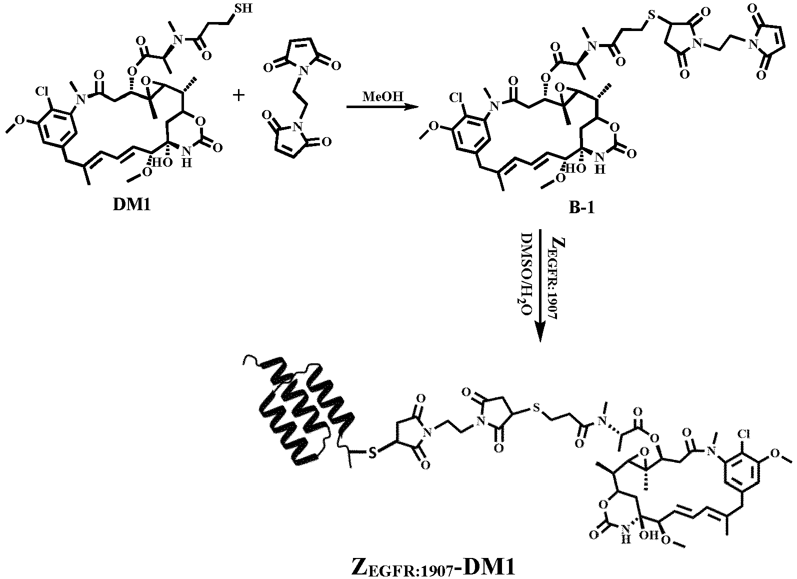

### 4.1 Synthesis of Conjugate Z_{EGFR:1907}-DM1

The affibody Z_{EGFR:1907} (12.3 mg) is dissolved in 5mL PBS solution, and then the intermediate B-1 (1.35 mg) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{EGFR:1907}-DM1 after the dialysis is completed.

The Maldi-Tof-MS spectrum of the conjugate Z_{EGFR:1907}-DM1 prepared by the embodiment are shown in Fig. 7, indicating that the single peak of the molecular weight of the product appears at 10382.53, which is basically consistent with the theoretical molecular weight of 10445.32.

The conjugate Z_{EGFR:1907}-DM1 prepared by the above is dissolved in dimethyl sulfoxide, and dripped into water at room temperature while removing dimethyl sulfoxide, so as to obtain a nanoparticle aqueous solution of the conjugate. An average size of the anti-tumor nanoparticle based on the conjugate Z_{EGFR:1907}-DM1 prepared in the embodiment is around 100nm.

### Embodiment 5

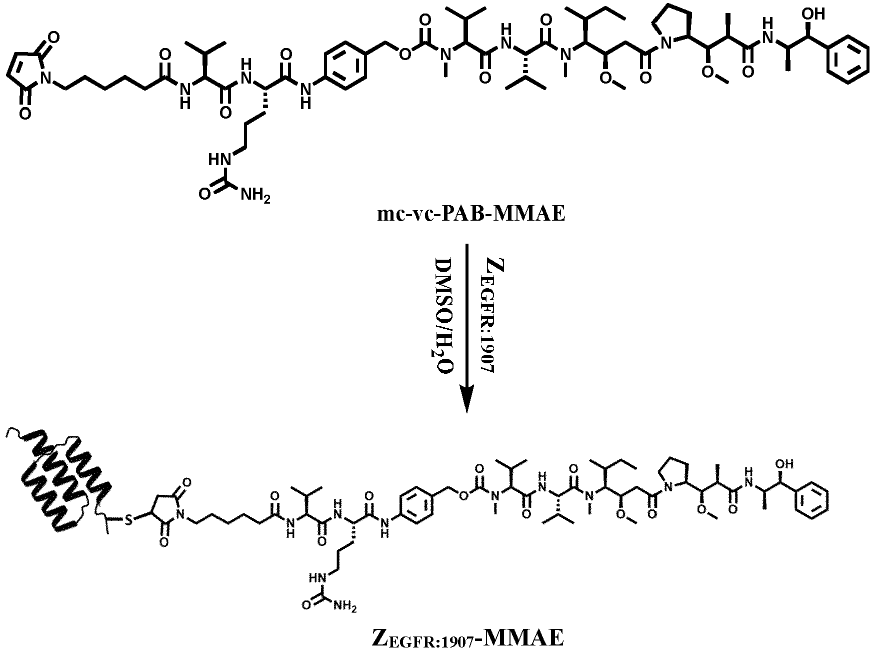

### 5.1 Synthesis of Conjugate Z_{EGFR:1907}-MMAE

The affibody Z_{EGFR:1907} (12.3 mg) is dissolved in 5mL PBS solution, and then an intermediate mc-vc-PAB-MMAE (1.85 mg) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{EGFR:1907}-MMAE after the dialysis is completed.

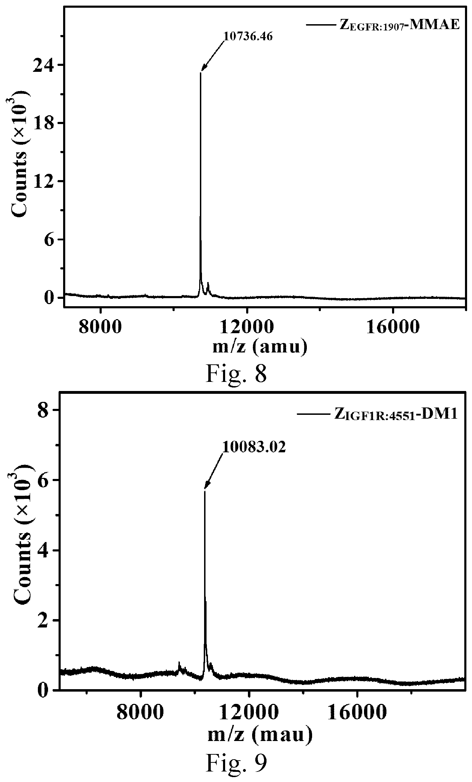

The Maldi-Tof-MS spectrum of the conjugate Z_{EGFR:1907}-MMAE prepared by the embodiment are shown in Fig. 8, indicating that the single peak of the molecular weight of the product appears at 10736.46, which is basically consistent with the theoretical molecular weight of 10803.53.

The conjugate Z_{EGFR:1907}-MMAE prepared by the above is dissolved in dimethyl sulfoxide, and dripped into water at room temperature while removing dimethyl sulfoxide, so as to obtain a nanoparticle aqueous solution of the conjugate. An average size of the anti-tumor nanoparticle based on the conjugate Z_{EGFR:1907}-MMAE prepared in the embodiment is around 100nm.

### Embodiment 6

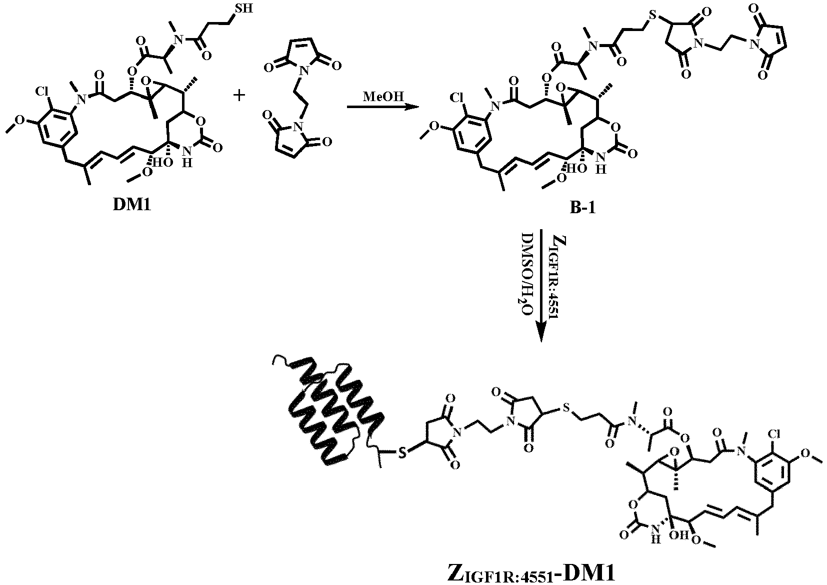

### 6.1 Synthesis of Conjugate Z_{IGF1R:4551}-DM1

The affibody Z_{IGF1R:4551} (12.8 mg) is dissolved in 5mL PBS solution, and then the intermediate B-1 (1.35 mg) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{IGF1R:4551}-DM1 after the dialysis is completed.

The Maldi-Tof-MS spectrum of the conjugate Z_{IGF1R:4551}-DM1 prepared by the embodiment are shown in Fig. 9, indicating that the single peak of the molecular weight of the product appears at 10083.02, which is basically consistent with the theoretical molecular weight of 10040.25.

The conjugate Z_{IGF1R:4551}-DM1 prepared by the above is dissolved in dimethyl sulfoxide, and dripped into water at room temperature while removing dimethyl sulfoxide, so as to obtain a nanoparticle aqueous solution of the conjugate. An average size of the anti-tumor nanoparticle based on the conjugate Z_{IGF1R:4551}-DM1 prepared in the embodiment is around 100nm.

### Embodiment 7

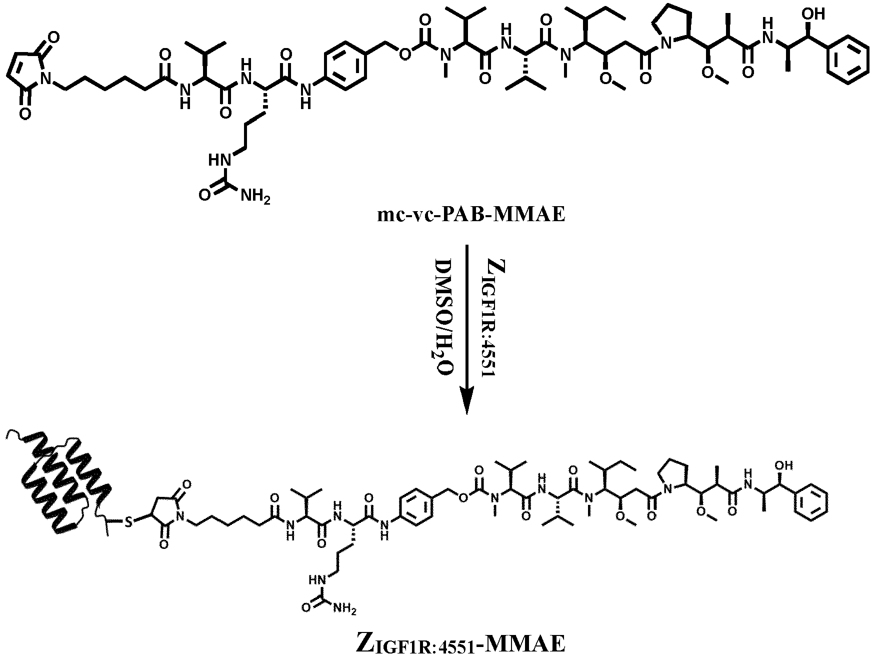

### 7.1 Synthesis of Conjugate Z_{IGF1R:4551}-MMAE

The affibody Z_{IGF1R:4551} (12.8 mg) is dissolved in 5mL PBS solution, and then the intermediate mc-vc-PAB-MMAE (1.85 mg) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{IGF1R:4551}-MMAE after the dialysis is completed.

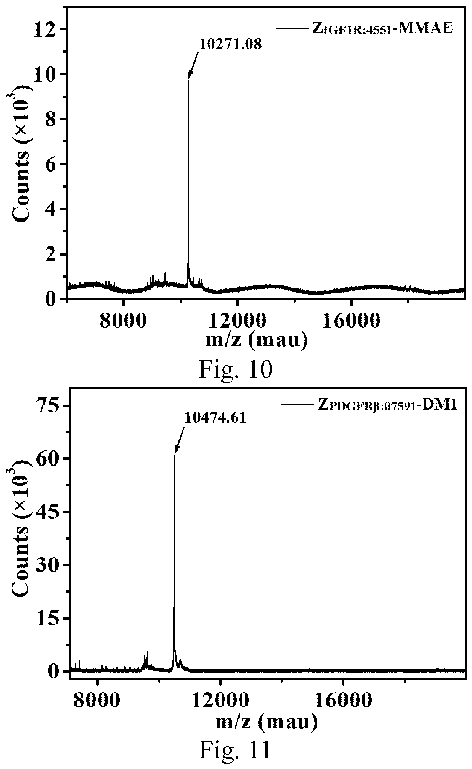

The Maldi-Tof-MS spectrum of the conjugate Z_{IGF1R:4551}-MMAE prepared by the embodiment are shown in Fig. 10, indicating that the single peak of the molecular weight of the product appears at 10271.08, which is basically consistent with the theoretical molecular weight of 10398.56.

The conjugate Z_{IGF1R:4551}-MMAE prepared by the above is dissolved in dimethyl sulfoxide, and dripped into water at room temperature while removing dimethyl sulfoxide, so as to obtain a nanoparticle aqueous solution of the conjugate. An average size of the anti-tumor nanoparticle based on the conjugate Z_{IGF1R:4551}-MMAE prepared in the embodiment is around 100nm.

### Embodiment 8

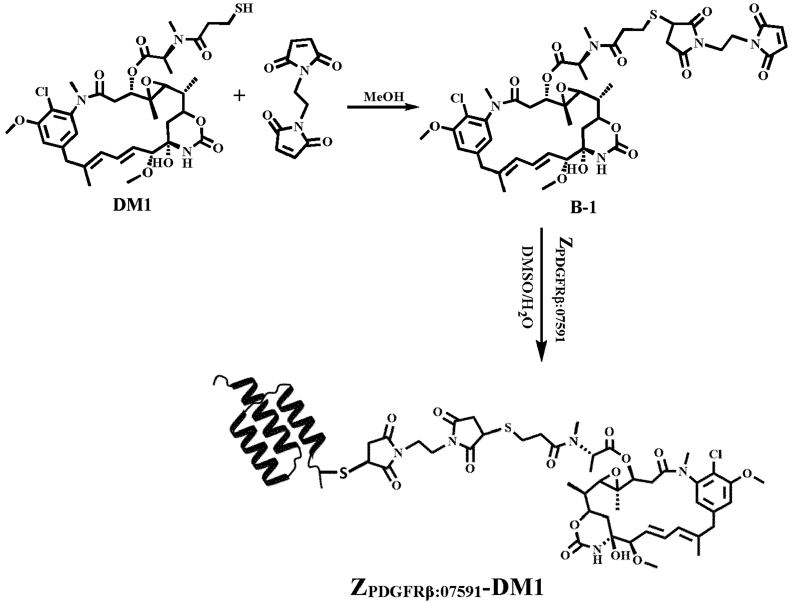

### 8.1 Synthesis of Conjugate Z_{PDGFRβ:07591}-DM1

The affibody Z_{PDGFRβ:07591} (13.4 mg) is dissolved in 5mL PBS solution, and then the intermediate B-1 (1.35 mg) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{PDGFRβ:07591}-DM1 after the dialysis is completed.

The Maldi-Tof-MS spectrum of the conjugate Z_{PDGFRβ:07591}-DM1 prepared by the embodiment are shown in Fig. 11, indicating that the single peak of the molecular weight of the product appears at 10474.61, which is basically consistent with the theoretical molecular weight of 10456.72.

The conjugate Z_{PDGFRβ:07591}-DM1 prepared by the above is dissolved in dimethyl sulfoxide, and dripped into water at room temperature while removing dimethyl sulfoxide, so as to obtain a nanoparticle aqueous solution of the conjugate. An average size of the anti-tumor nanoparticle based on the conjugate Z_{PDGFRβ:07591}-DM1 prepared in the embodiment is around 100nm.

### Embodiment 9

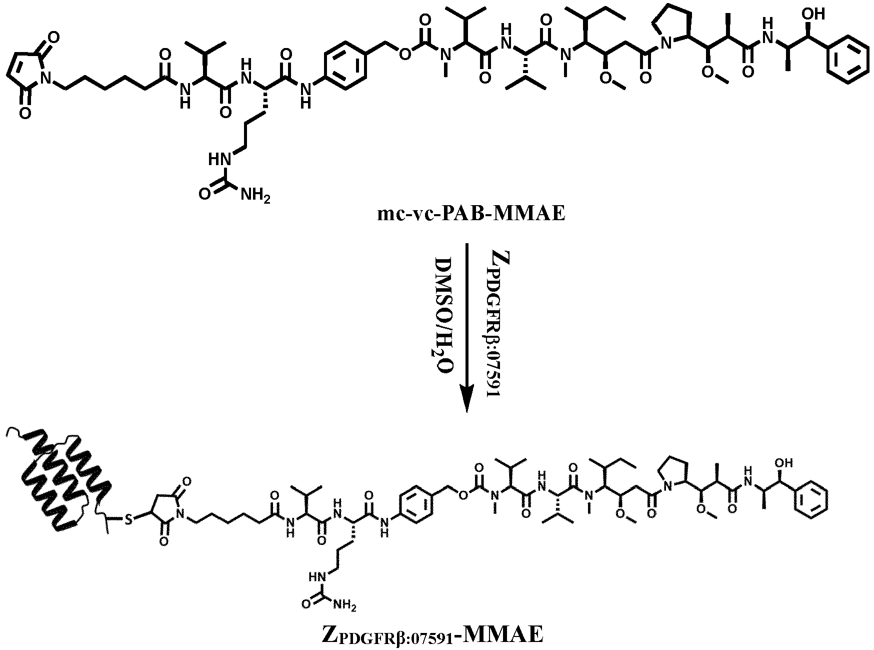

### 9.1 Synthesis of Conjugate Z_{PDGFRβ:07591}-MMAE

The affibody Z_{PDGFRβ:07591} (13.4 mg) is dissolved in 5mL PBS solution, and then the intermediate mc-vc-PAB-MMAE (1.85 mg) is dissolved in 150µE dimethyl sulfoxide and slowly added dropwise to the above affibody solution, which are stirred for reaction for 12 hours at room temperature. The reaction solution is purified by dialysis in water, and freeze-dried to obtain the white cotton-like conjugate Z_{PDGFRβ:07591}-MMAE after the dialysis is completed.

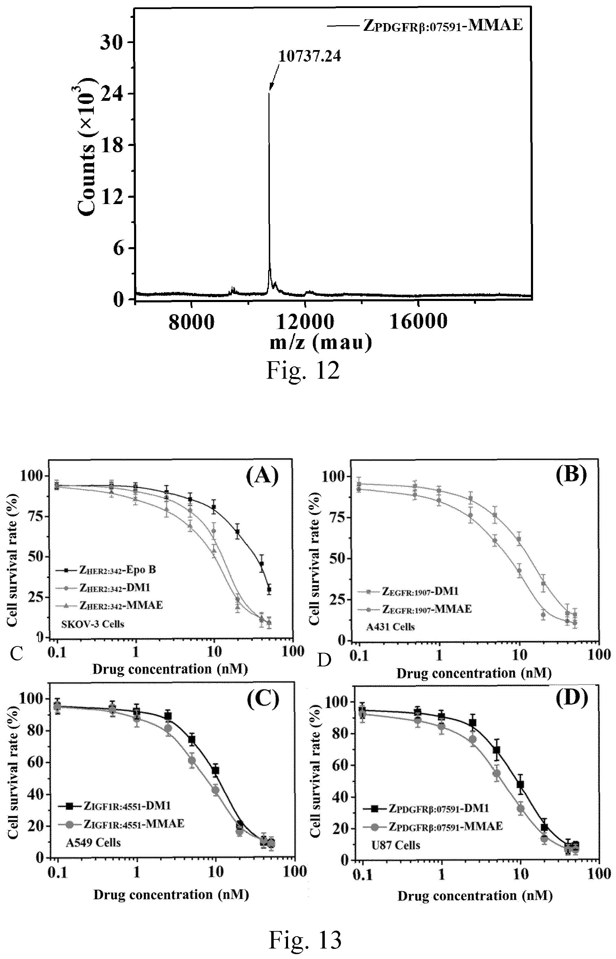

The Maldi-Tof-MS spectrum of the conjugate Z_{PDGFRβ:07591}-MMAE prepared by the embodiment are shown in Fig. 12, indicating that the single peak of the molecular weight of the product appears at 10737.24, which is basically consistent with the theoretical molecular weight of 10815.03.

The conjugate Z_{PDGFRβ:07591}-MMAE prepared by the above is dissolved in dimethyl sulfoxide, and dripped into water at room temperature while removing dimethyl sulfoxide, so as to obtain a nanoparticle aqueous solution of the conjugate. An average size of the anti-tumor nanoparticle based on the conjugate Z_{PDGFRβ:07591}-MMAE prepared in the embodiment is around 100nm.

### Embodiment 10

The embodiment provides an experiment on the effect of the nanoparticle of the affibody-cytotoxin conjugate for active targeted therapy of tumors.

The nanoparticles of the affibody-cytotoxin conjugate prepared in Embodiments 1 to 9 are prepared into solutions with concentrations of 0.1, 0.5, 1, 2.5, 5, 10, 20, 40, and 50 nmol/L with cell culture medium, and then cultured with SKOV-3 cells (ovarian cancer cell line), A431 cells (surface skin cancer cell line), A549 cells (lung cancer cell line) and U87 cells (brain glioma cell line) for 48 hours, followed by performing a test on cell viability using the MTT method, leading to results shown as A, B, C and D in Fig. 13.

When the concentration of the nanoparticle of the affibody-cytotoxin conjugate reaches 10 nmol/L, the proliferation of cancer cells is significantly inhibited, and the inhibitory effect of the nanoparticle of the affibody-cytotoxin conjugate on cancer cells is proportional to the concentration. This indicates that the nanoparticles of the affibody-cytotoxin conjugate prepared in Embodiments 1 to 9 have potential applications in the treatment of malignant tumors.

### Embodiment 11

The embodiment provides a pharmacokinetic experiment of the nanoparticle of the affibody-cytotoxin conjugate Z_{HER2:342}-Epo B for active targeted therapy of tumors in SD rats.

With Cy5.5 (cyanine dye 5.5) as a fluorescent probe molecule in vivo, Cy5.5 is covalently modified on the surface of the nanoparticle of the conjugate with active targeting function Z_{HER2:342}-Epo B obtained in Embodiment 1 for evaluation of the in vivo pharmacokinetic properties of the nanoparticle.

Two SD rats (-200 g) are randomly selected as an experimental group, and injected with 200 µL of Z_{HER2:342}-Epo B nanoparticles covalently modified by Cy5.5 into the tail vein respectively; at the set time points (15 min, 30 min, 1 h, 2 h, 4 h, 8 h, and 12 h), about 0.5 mL of blood is taken from the mouse orbit into a test tube containing sodium heparin, and then stored at 4 °C refrigerator. After all the data points are collected, the obtained blood samples are centrifuged at 3000 rpm for 10 min to obtain upper serum samples. 200 µL of serum is taken at each time point, then the content of Cy5.5 is analyzed by fluorescence spectroscopy, and then the content of corresponding Z_{HER2:342}-Epo B is obtained after conversion

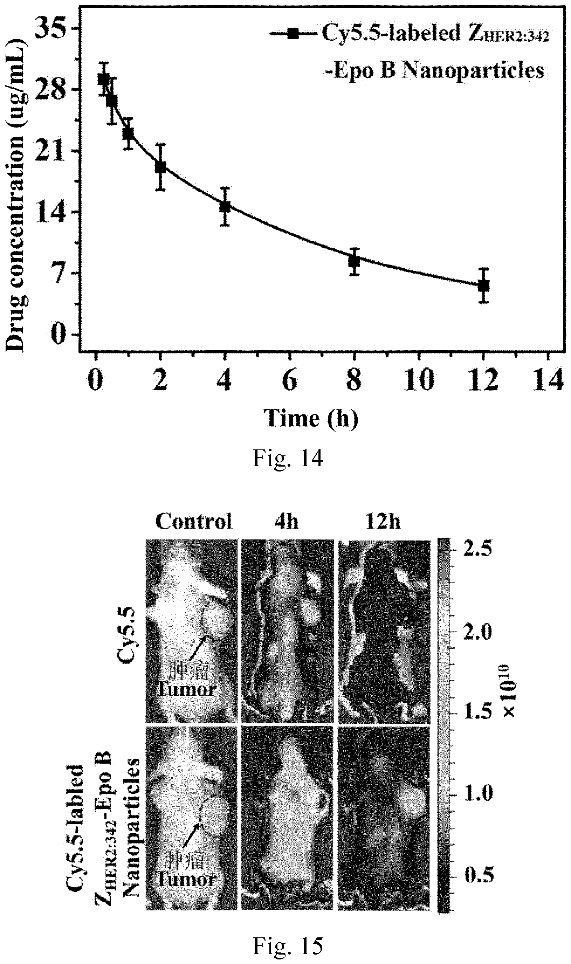

The results are shown in Fig. 14. It can be inferred from the figure that a blood half-life period of the Z_{HER2:342}-Epo B nanoparticle is about 6 h, which is much higher than the blood half-life period of the conventional small-molecule anti-tumor drug (for example, paclitaxel, a front anti-cancer drug in clinical chemotherapy, has a blood half-life period of only 1.1 h in rats). 12 h after tail vein injection of the Z_{HER2:342}-Epo B nanoparticle in rats, the Z_{HER2:342}-Epo B nanoparticle in the blood still maintains a high concentration of 5.6 µg/mL, which indicates that the Z_{HER2:342}-Epo B nanoparticle may be effectively retained in the blood circulation system; a longer residence time in blood is also conducive to more enrichment of the Z_{HER2:342}-Epo B nanoparticle in tumor sites, thereby achieving effective anti-tumor effects.

### Embodiment 12

The embodiment provides a diagram of long-term cycle imaging of the nanoparticle of the affibody-cytotoxin conjugate Z_{HER2:342}-Epo B for active targeted therapy of tumors in tumor-bearing nude mice.

With Cy5.5 (cyanine dye 5.5) as a fluorescent probe molecule in vivo, Cy5.5 is covalently modified on the surface of the nanoparticle of the conjugate with active targeting function Z_{HER2:342}-Epo B obtained in Embodiment 1 for evaluation of the in vivo circulation time and the targeting effects of nanoparticle. A cell suspension of SKOV-3 cells in logarithmic growth phase is prepared at a concentration of 4.0×10⁶ cells/mL, and inoculated subcutaneously into the right anterior axillary of nude mice, 200 µL per mouse; when the volume of tumor is around 500 mm³, two mice are randomly selected as a control group and an experimental group. For the experimental group, 200 µL of the Z_{HER2:342}-Epo B nanoparticle covalently modified by Cy5.5 is injected; for the control group, 200 µL of Cy5.5 solution is injected (the content of Cy5.5 in the solution of the experimental group is the same as that in the solution of the control group). After 4h and 12h of injection, ZKKS-Mulaurora imaging system is used to take pictures and analyze the fluorescence intensity of the whole body of the mice.

The results are shown in Fig. 15, the small molecule Cy5.5 has no obvious presence in the mouse body after 4 hours of injection, and is basically completely metabolized after 12 hours; meanwhile, the ZHER2:342-Epo B nanoparticle is significantly enriched in the tumor site after 4 hours of injection without obvious presence in other organs, and after the nanoparticle is circulated for 12 hours in mice, obvious fluorescent signals may still be observed at the tumor site. The results show that the nanoparticle of the affibody-cytotoxin conjugate has excellent tumor targeting properties and may circulate in vivo for a long time, which has potential application value in tumor therapy.

### Embodiment 13

The embodiment provides a diagram showing therapeutic effects on tumors of tumor-bearing mice treated with the nanoparticle of the affibody-cytotoxin conjugate Z_{HER2:342}-MMAE for active targeted therapy of tumors.

A cell suspension of SKOV-3 cells in logarithmic growth phase is prepared at a concentration of 4.0×10⁶ cells/mL, and inoculated subcutaneously into the right anterior axillary of nude mice, 200 µL per mouse; when the volume of tumor is around 100 mm³, the mice are randomly selected into 4 groups, 5 in each group, which are respectively a PBS control group, a MMAE 0.6 mg/kg group, a Z_{HER2:342}-MMAE nanoparticle group (the content of MMAE is 0.4 mg/kg), and a Z_{HER2:342}-MMAE nanoparticle group (the content of MMAE is 0.6 mg/kg), wherein 200 µl PBS is injected for the PBS group, 0.6 mg/kg of MMAE is injected for the MMAE 0.6 mg/kg group, Z_{HER2:342}-MMAE (containing 0.4 mg/kg MMAE) is injected for the Z_{HER2:342}-MMAE group (containing 0.4 mg/kg MMAE), and Z_{HER2:342}-MMAE (the content of MMAE is 0.6 mg/kg) is injected for the Z_{HER2:342}-MMAE group (the content of MMAE is 0.6 mg/kg), once every 3 days. After each administration, the survival of tumor-bearing nude mice is observed, and the body weight and tumor size of the mice are measured and photographed for preservation.

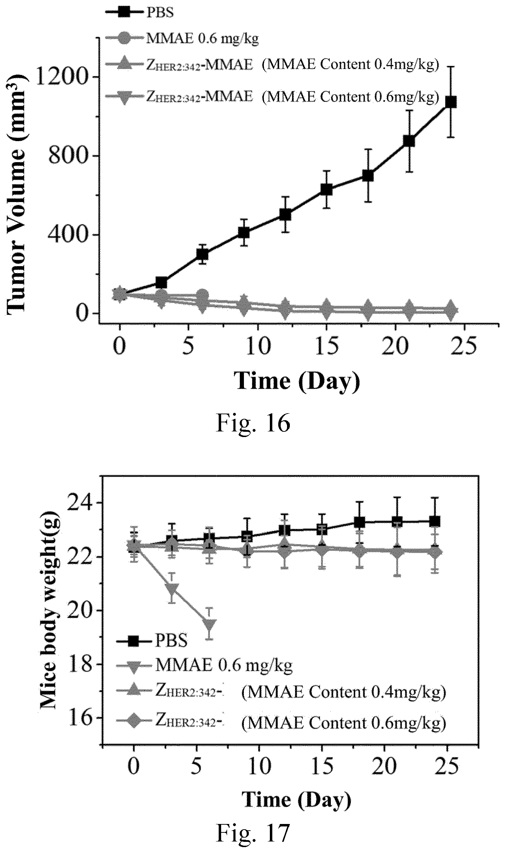

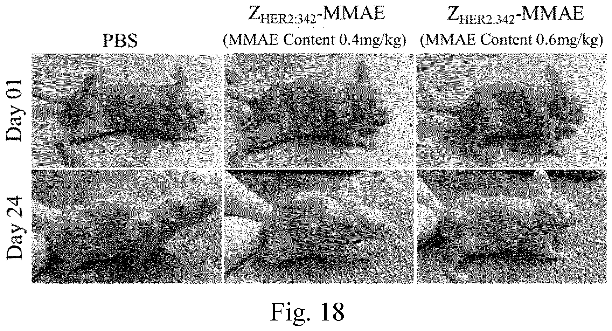

The experimental results are shown in Figs. 16, 17 and 18, the tumors of the mice in the PBS control group grow rapidly; the body weight of the mice in the MMAE 0.6 mg/kg group drops sharply after administration, and finally all died after 3 administrations, which indicates that the MMAE raw drug has strong toxic side effects; meanwhile, after the tumor-bearing mice are treated with the Z_{HER2:342}-MMAE nanoparticle (the content of MMAE is 0.4 mg/kg), the tumor volume is significantly reduced, and after the dose is increased to 0.6 mg/kg, the tumor volume of tumor-bearing mice is significantly reduced until it almost disappears while the survival condition being good.

These results show that the targeting property of the nanoparticle of the affibody-cytotoxin conjugate Z_{HER2:342}-MMAE not only brings excellent anti-tumor effect in vivo, but also effectively reduces the toxic side effects of NMAE. The excellent anti-tumor effects and good biological safety prove that the affibody-cytotoxin conjugate is a new formulation of antibody-drug conjugate with great market prospects.

The above disclosure is only the preferred embodiment of the present invention. The preferred embodiments do not describe all the details, and are not intended to limit the invention only to be the specific embodiments. It is obvious that various modifications and changes can be made to the content of the specification. The present invention selects and specifically describe the embodiments with the purpose of better explain the principle and practical use of the present invention, such that a person skilled in the art can well utilize the present invention. The present invention is merely limited by the appended claims and the scope and equivalents thereof.

Under the teaching of the invention and the above-mentioned embodiments, those skilled in the art can easily foresee that each raw material listed or exemplified in the invention or its equivalent replacement and each processing method or its equivalent replacement may all realize the invention, and the upper and lower limit values and interval values of the parameters of each raw material and processing method may realize the invention. Embodiments are not enumerated here one by one.

## Claims

1. A affibody-cytotoxin conjugate for active targeted therapy of tumors, mainly comprising a cytotoxin and an affibody with active targeting function, the conjugate being obtained by covalent coupling the cytotoxin and the affibody through a small molecule linker; 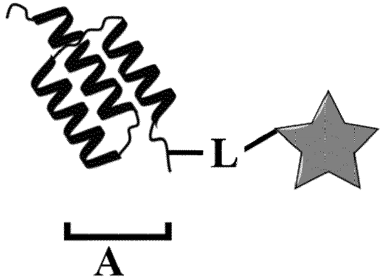 wherein A is the affibody, L is the organic small molecule linker, and 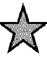 is the cytotoxin.

2. The affibody-cytotoxin conjugate for active targeted therapy of tumors according to claim 1, wherein the affibody is selected from at least one of an affibody targeting human epithelial growth factor receptors Z_{HER2:342} or Z_{EGFR:1907}, an affibody targeting human insulin-like growth factor receptors Z_{IGF1R:4551}, and an affibody targeting human platelet-derived growth factor receptors β Z_{PDGFRβ:07591}.

3. The affibody-cytotoxin conjugate for active targeted therapy of tumors according to claim 1, wherein the cytotoxin is selected from at least one of epothilone B and derivatives thereof, maytansine and derivatives thereof, and monomethyl auristatin E and derivatives thereof.

4. The affibody-cytotoxin conjugate for active targeted therapy of tumors according to claim 1, wherein the small molecule linker is selected from at least one of formula (II), formula (III) and formula (IV) as below; 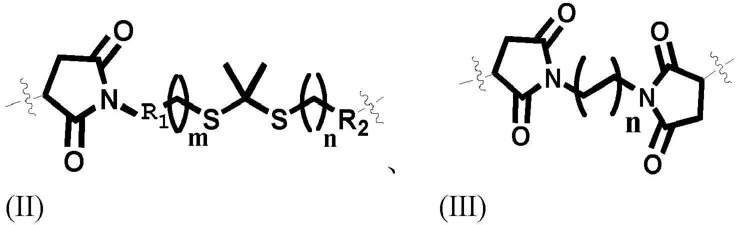 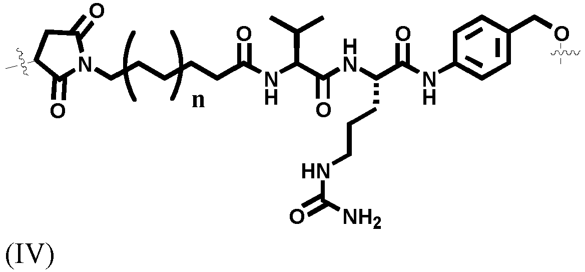
wherein R1 is selected from -alkyl-C(O)NH-, -alkyl-SO₂NH-, or C1-8 alkoxy; R2 is selected from carboxyl or acid chloride, and n and m are each independently selected from 2 to 10;
the alkyl is selected from C1-8 alkyl; the alkyl group and alkoxy group are unsubstituted or substituted by 1, 2, 3 substituents selected from the group consisting of halogen, cyano, acetyl, hydroxy, hydroxymethyl, hydroxyethyl, carboxyl, C1-8 alkyl, C1-8 alkoxy, halogenated C1-8 alkyl, C3-8 cycloalkyl, C3-8 cycloalkoxy, Halogenated C1-8 alkoxy, -C(O)C1-10 alkyl, -C(O)OC1-10 alkyl, -OC(O)C1-10 alkyl, -CONRₐ₀R_{b0}; Rₐ₀ and R_{b0} are each independently hydrogen or C1-8 alkyl.

5. The affibody-cytotoxin conjugate for active targeted therapy of tumors according to claim 1, wherein the conjugate is one of the following structures: 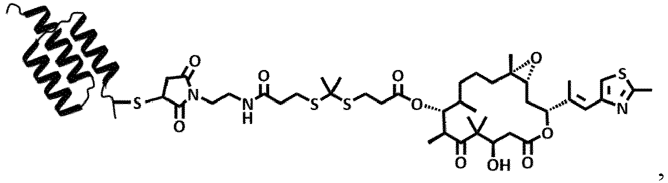 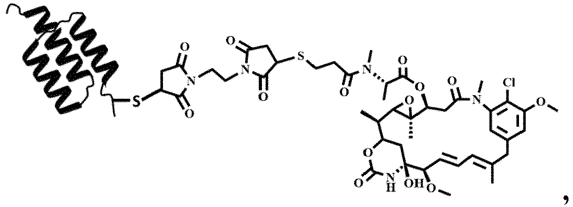 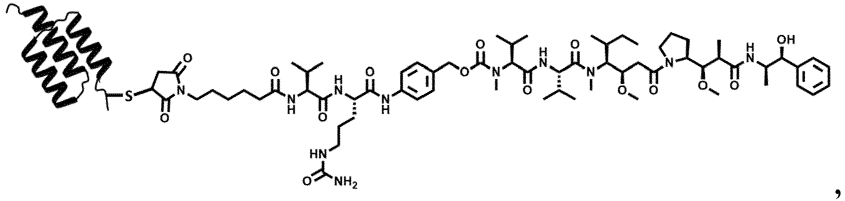 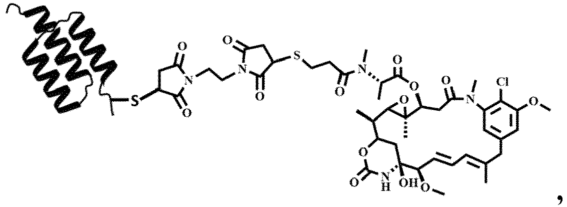 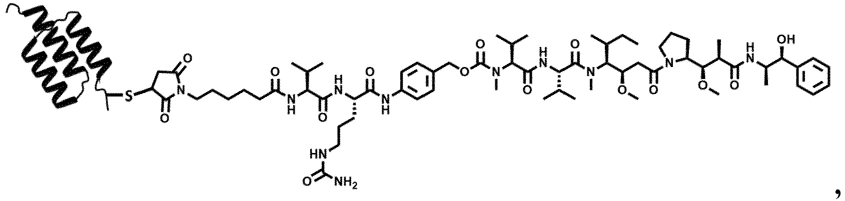 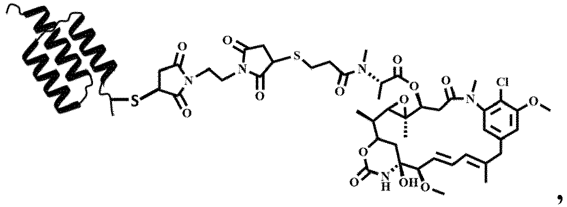 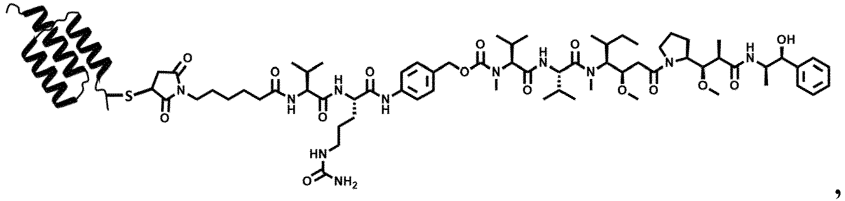 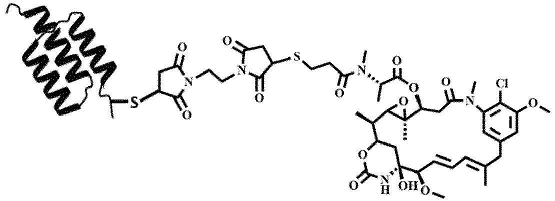 or

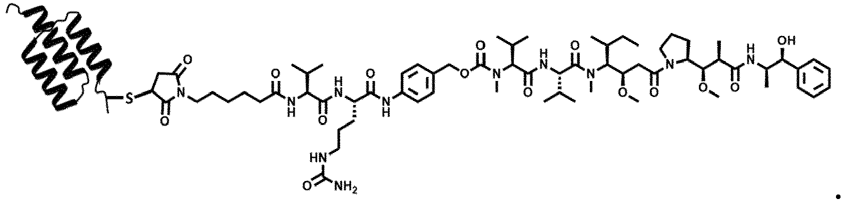

6. A nanoparticle of the affibody-cytotoxin conjugate for active targeted therapy of tumors, obtained by self-assembly of the conjugate according to any one of claims 1 to 5 in an aqueous medium.

7. The nanoparticle according to claim 6, wherein a shell of the nanoparticle is composed of the affibody with active targeting function, an inner core of the nanoparticle is composed of the small molecule linker and the cytotoxin, and a particle size of the nanoparticle is less than 200 nm.

8. A preparation method for the nanoparticle of the affibody-cytotoxin conjugate for active targeted therapy of tumors, comprising steps of:
(1) bonding the hydrophobic cytotoxin containing hydroxyl and/or amino and/or sulfhydryl to the small molecule linker, which is further subjected to conjugation reaction with an active targeting affibody, so as to obtain the affibody-cytotoxin conjugate with active targeting function;
(2) dissolving a certain amount of the affibody-cytotoxin conjugate in the step (1) in a certain volume of an organic solvent, which is dripped into slowly-stirring ultrapure water at room temperature, and removing the organic solvent, so as to obtain a nanoparticle aqueous solution of the conjugate with active targeting function.

9. The preparation method according to claim 8, wherein the organic solvent in the step (2) is selected from at least one of dimethyl sulfoxide, N,N'-dimethylformamide, ethanol, and isopropanol.

10. An application of the affibody-cytotoxin conjugate for active targeted therapy of tumors according to any one of claims 1 to 5, or the nanoparticle according to claim 6 or 7 or the nanoparticle prepared by the preparation method according to claim 8 or 9 in preparing drugs for active targeted therapy of tumors.
